(19) Europäisches Patentamt
European Patent Office
Office européen des brevets

(11) EP 1 479 665 A1

(12) EUROPEAN PATENT APPLICATION

(43) Date of publication:
**24.11.2004 Bulletin 2004/48**

(51) Int Cl.[7]: **C07C 29/04**, B01J 31/10, B01J 37/06

(21) Application number: **04252612.9**

(22) Date of filing: **05.05.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **20.05.2003 US 471912 P**

(71) Applicant: **ROHM AND HAAS COMPANY**
**Philadelphia, Pennsylvania 19106-2399 (US)**

(72) Inventors:
- **Collin, Jennifer Reichl**
**Devon Pennsylvania 19333 (US)**
- **Ramprasad, Dorai**
**Allentown Pennsylvania 18104 (US)**

(74) Representative: **Kent, Venetia Katherine et al**
**Rohm and Haas (UK) Ltd**
**European Patent Department**
**28th. Floor, City Point**
**One Ropemaker Street**
**London EC2Y 9HS (GB)**

(54) **Methods, systems and catalysts for the hydration of olefins**

(57) The present invention provides a system, method and catalyst for olefin hydration. The method includes hydrating the olefin using a base treated, sulfonated, halogenated and acid regenerated thermally stable catalyst. In several variants, the olefin hydration comprises butene hydration, propene hydration, hydration of cyclohexene, propylene hydration, pentene hydration, hexene hydration, and heptene hydration. The present invention also provides a method of making a catalyst for olefin hydration, and provides alcohols manufactured by the catalyst(s), systems and methods described herein.

EP 1 479 665 A1

## Description

This invention relates to a method of, and system and catalyst for olefin hydration. The invention also provides for a method of making the catalyst capable of olefin hydration, and a catalyst composition.

BACKGROUND OF INVENTION

The use of sulfonated polystyrene resins crosslinked with divinyl benzene as catalysts for the hydration of olefins such as propylene or butene has been generally described in the literature. See, Hydrocarbon Processing, Nov. 1988, pp75-78 and US 4,579,984 and references therein. Olefins such as n-butene have a limited solubility in water and are less reactive necessitating elevated temperatures such as 150-170 degrees C. However, the use of elevated temperatures has the significant drawback of a resulting loss of $SO_3H$ groups reducing the catalyst activity and corroding a reactor. There exists a need for thermally stable catalysts capable of olefin hydration at high temperatures.

A major problem of all of these attempts in the prior art is corrosion, and very long reaction times. Chlorinated polymers can split off HCl in addition to sulfuric acid during use and can lead to corrosion of the stainless steel reactor. US Patent No. 4,705,808 generally relates to a process for pretreatment with deionized water for 400 hours in the absence of oxygen or metal ion at 100-150 degrees C under pressure. This art still has the drawback of requiring significant reaction times. There exists a need in the art for a thermally stable catalyst, method and system that solves the corrosion problem and provides for greatly reduced reaction times.

There exists a significant need in the art for methods of olefin hydration, and catalysts that can be used for olefin hydration that are thermally stable and provide improved performance characteristics. In addition to solving other problems in the art, the present invention provides solutions to the problems mentioned above and satisfies the needs of the art.

In one aspect, the present invention relates to a method of olefin hydration using a catalyst that includes a styrene DVB copolymer, and/or a DVB and EVB copolymer. The method includes hydrating the olefin using a base treated, sulfonated, halogenated and acid regenerated thermally stable catalyst. In several variants, the olefin hydration comprises butene hydration, cyclohexane hydration, and propene hydration. That is, in one variant of the invention the following is accomplished: hydration of propene to isopropanol, hydration of cyclohexene to cyclophexanol, and hydration of 1-butene to secondary butyl alcohol.

In yet a further aspect, the invention provides an improved styrene DVB resin catalyst comprising 4-chlorostyrene aromatic groups and a polymer backbone. At least one of the aromatic groups has a chlorine in a styrenic para-position, and optional other chlorines thereon. The polymer backbone is substantially free of leachable chlorine. The catalyst includes a halogenated DVB moiety. In one variant, the aromatic rings are oleum sulfonated. In yet another variant, the styrene DVB resin further comprises sulfone bridges. Another variant includes a 4-halo (F,Br, and/or I) styrene.

The present invention relates to a method of olefin hydration using a catalyst that includes a styrene DVB copolymer and/or DVB/EVB copolymer. The method includes hydrating the olefin using a base treated, sulfonated, halogenated and acid regenerated thermally stable catalyst. In several variants, the olefin hydration comprises butene hydration: hydration of 1-butene to secondary butyl alcohol; propene hydration: hydration of propene to isopropanol; and, cyclohexane hydration: hydration of cyclohexene to cyclohexanol. The method can also be used to hydrate propylene, pentene, hexene and/or heptene. It is also appreciated that the method is suitable for hydration of olefins other than those listed herein, and derivatives thereof. The production of lower alcohols such as butyl or propyl alcohols are among the most commercially significant operations undertaken in the chemical industry. The principle means by which these alcohols, and other alcohols are manufactured is through the hydration of olefins. Use of the catalysts, methods and systems described herein provides unexpected performance improvements in olefin hydration.

In another aspect, the invention provides a method of making a thermally stable catalyst for olefin hydration. The method includes base treating a sulfonated and halogenated copolymer to obtain a base treated copolymer, and regenerating the base treated copolymer with an acid. In another variant, the method of making a thermally stable catalyst for olefin hydration includes base treating a halogenated copolymer to obtain a base treated copolymer, and sulfonating the base treated copolymer.

In yet another variant, leachable chlorine is removed from a polymer backbone of the catalyst by the base treatment. Manufacture of the catalyst described herein has the advantage of reducing the level of corrosion of equipement used for olefin hydration versus conventional catalysts since there is significantly reduced leachable chlorine on the polymer backbone of the catalyst described herein. It is appreciated that significant cost savings are achieved and equipment longevity is increased through the use of the method, catalyst, and systems that use the method and catalyst described herein. Moreover, in one variant of the invention a polymer backbone is substantially free of leachable chlorine when there are no or very limited corrosion effects on equipment and/or prolonged life of the equipment as a result of use of the catalyst described herein.

In yet a further variant, the method of making a thermally stable catalyst for olefin hydration includes base treating a sulfonated and halogenated copolymer to obtain abase treated copolymer, and regenerating the base treated copolymer with an acid. The method of making a thermally stable catalyst for olefin hydration optionally includes chlorinating a styrene DVB copolymer, base treating the halogenated copolymer, and sulfonating the halogenated and base treated catalyst. The styrene DVB copolymer is a polysulfonated copolymer in another variant of the invention.

In yet a further aspect, the invention provides an improved styrene DVB resin catalyst comprising aromatic groups having more than one $SO_3H$ moiety and a polymer backbone. The improvement includes halogenated aromatic groups. The polymer backbone is substantially free of leachable chlorine. The catalyst is capable of olefin hydration. In one variant, the leachable chlorine is at least an order of magnitude less than the leachable chlorine of a catalyst not base treated. In yet a further variant, the polymer backbone of the catalyst is free of leachable chlorine or other detrimental halogens.

In yet a further aspect, the invention provides an improved styrene DVB resin catalyst comprising 4-chlorostyrene or any 4-halostyrene aromatic groups and a polymer backbone. At least one of the aromatic groups has a chlorine or other halogen in a styrenic para-position, and optional other chlorines or halogens thereon. The polymer backbone is substantially free of leachable chlorine or other halogens. The catalyst includes a halogenated DVB moiety. In one variant, the aromatic rings are oleum sulfonated. In yet another variant, the styrene DVB resin further comprises sulfone bridges.

In yet a further aspect, the invention provides a method of reducing the compressibility of a styrene DVB monosulfonated catalyst. The method includes increasing the number of chlorine on aromatic rings of the catalyst as compared to unhalogenated catalysts. In yet a further variant of the invention, the method of olefin hydration utilizes a base treated, sulfonated, chlorinated and acid regenerated thermally stable catalyst.

Variants of the invention relate to preparation of resins of high thermal stability with low leaching of chlorine which are used as catalysts with high activity for olefin hydration, e.g. propene and/or butene hydration. Several types of catalytic resins were prepared according to the reaction schemes shown below.

The first reaction scheme for making a catalyst of the present invention involves the following process:

Styrene DVB copolymer --→ Sulfonate -→ chlorinate (or halogenate) -→ base treat --→

regenerate with acid.

The chlorination (or halogenation) step incorporates chlorine (or other halogen) in the aromatic ring as well as the aliphatic backbone of the polymer. It is the chlorine (or other halogen) on the backbone that can undesireably leach of slowly as HCl (or other corrosive compound) in an olefin hydration process. Where this happens, accelerated equipment corrosion occurs resulting in undesirable down time and equipement replacement costs. Heating the polymer in a basic solution results in, for example, the leachable chlorine being removed. Moreover, this process can be carried out in the space of a several hours in contrast to the art process which takes 10 days to carry out, e.g. US Patent No. 4,705,808. It is appreciated that the time needed to manufacture a catalyst of the invention is greatly reduced to the range of several hours, e.g. 1-10 hours in one variant of the invention.

The final polymer is created using the process that has a higher acid site density than that created by prior methodologies. The current process does not have the drawback of the known processes since there is no extensive desulfonation in addition to chlorine removal. Consequently, a catalyst created by the process used herein has the advantage of lower desulfonation than catalysts produced by conventional processes. This results in higher catalytic activity since more active sites remain on the catalyst than after preparation using conventional processes.

Scheme 1 was used to chlorinate Amberlyst 39, a commercially available sulfonated styrene DVB(7%) copolymer from Rohm & Haas Company. Other suitable resins could also be used herein. After refluxing with a 2N sodium hydroxide solution for 22 hours, followed by regeneration with hydrochloric acid, a new resin(**A**) was obtained. The hydrolytic stability of this resin was tested at 200C for 24 hours. Table 1 shows that Resin A loses 9.5% of its sulfonic acid groups versus 33.4% lost by resin F which is a conventional chlorinated resin containing 12% DVB. Additionally, the percentage chlorine detected in solution is much less for resin **A**. We also subjected resin **F** to a base treatment to get resin **B** which has somewhat improved stability and lower chlorine leaching than resin **F**. Interestingly, resin **F** was also post treated using the process described in US Patent No. 4,705,808 to get resin **C**. It is clear from Table 1 that resin **B** has a higher acid capacity and is different from resin **C** in its properties and performance characteristics. The variant of the invention of scheme 1 involves a chlorination step followed by base treatment prior to the sulfonation. Additionally, the base treatment could be performed in any other solvent or mixtures of solvents. The optimum conditions are resin specific, and can readily be determined empirically. In the examples described , we have described the use of a 50% NaOH solution for 4 hrs at 130 degrees **C**.

Scheme 2 is also used to obtain a catalyst of the present invention:

Styrene DVB copolymer --→ polysulfonate using oleum -→ chlorinate (or otherwise

halogenate)-→ base treat --→ regenerate with acid.

Oleum sulfonation of a styrene DVB co-polymer results in a polysulfonated polymer ( one that has greater than one $SO_3H$ group per ring). It is appreciated that the resin created by the process is both a polysulfonated and a chlorinated resin. Amberlyst 36 is a polysulfonated resin with 12% DVB. This resin was chlorinated and base treated as in Scheme 2 to get resin D. The thermal stability results of resin D (see Table 1) shows that it has high acid capacity, good stability and low chlorine leaching.

Scheme 3 is also used to obtain a catalyst of the present invention:

4-chlorostyrene DVB copolymer -→ sulfonate using oleum -→ chlorinate (or otherwise

halogenate)-→ base treatment -→ regenerate with acid

A monomer is prepared by British Patent 1,393,594. A halogenated monomer such as chlorostyrene with a crosslinker such as divinylbenzene is used, thereafter one sulfonates the polymer using chlorosulfonic acid. This polymer is still unstable at high temperatures because the DVB part is not deactivated by a halogen atom. In this variant of the invention, this polymer is chlorinated even further creating very desirable catalytic properties as well as solving the temperature stability problem by having high termperature stability. While not being bound by theory, we hypothesize that a chlorination of the DVB creates a very stable resin since all the $SO_3H$ groups on the chlorinated resin will be in a meta position.

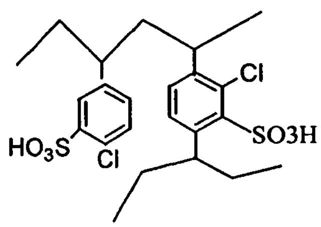

In another variant of the invention, a copolymer of 4-chlorostyrene with 12% DVB was prepared and then monosulfonated using oleum as the sulfonating agent for relatively short reaction times. This is a substantial improvement on the new procedure described in British Patent 1,393,594. The resulting resin has sulfone bridges which increase thermal stability. The resin was then further chlorinated and then base treated to remove leachable chlorine and after acid regeneration yielded resin **E**. The thermal stability of this resin and the low chlorine leaching coupled with the high acid capacity provides the resin with important catalytic properties and unexpected performance characteristics. Scheme 3 can therefore be used to prepare a variety of styrene DVB polymers that are fully halogenated and have $SO_3H$ groups in a meta position. This method can be also used to prepare polysulfonated chlorinated resins as in Scheme 2 by varying the sulfonation conditions. In addition to this, the chlorination of the copolymer and base treatment can be performed prior to the sulfonation step.

It is further appreciated that the steps described in the various schemes mentioned above can be combined in various permutations such that the desired catalytic properties of the catalyst are obtained herein.

TABLE 1

| RESIN | %Cl %S | MmolSO3H/g | Mmol SO3H/g after 200C, | % loss in acid sites | %chlorine in liquid after |
|---|---|---|---|---|---|
| | | | 24hours | | 200C, 24 hours |
| **A** | 26.78, 8.62 | 2.75 | 2.49 | 9.45 | 0.002 |
| **B** | 19.62, 10.24 | 3.31 | 2.50 | 24.60 | 0.19 |
| **F** | 22.00 | 3.14 | 2.10 | 33.40 | 1.34 |

TABLE 1 (continued)

| RESIN | %Cl %S | MmolSO3H/g | Mmol SO3H/g after 200C, | % loss in acid sites | %chlorine in liquid after |
|---|---|---|---|---|---|
| | | | 24hours | | 200C, 24 hours |
| **C** | 19.30 | 3.06 | 2.12 | 30.40 | 0.08 |
| **D** | 10.04, 16.69 | 4.78 | 3.79 | 20.70 | 0.11 |
| **E** | 15.96, 13.95 | 4.16 | 3.52 | 15.34 | 0.12 |

COMPRESSIBILITY OF RESINS

Chlorination (or other halogenation) of a monosulfonated styrene DVB resin reduces its compressibility. The change in the compressibility depends on the percent DVB in resin and on the degree of chlorination or other halogenation. The results are shown below.

| RESIN | COMPRESSIBILITY cm/m |
|---|---|
| Styrene 12% DVB copolymer monosulfonated | 4.03 |
| Styrene 12% DVB copolymer chlorinated to 20% by weight | 3.03 |
| Styrene 7% DVB copolymer monosulfonated | 7.23 |
| Styrene 7% DVB copolymer monosulfonated and chlorinated to 27% by weight | 4.39 |

CATALYTIC ACTIVITY OF RESINS

Resins **A-E** were tested as catalysts for the hydration of 1-butene to sec-butyl alcohol. Both a flow system and a batch reactor can be systems used in the present invention. Though the industrial process is run in a flow system, the catalytic activity was performed in a batch reactor. The conditions used were 155 degrees C and 1000 psig to imitate the supercritical conditions of the industrial process. The reaction was stopped after one hour and the product analyzed. It was found that the conversions were between 3-4% and the selectivity to sec-butyl alcohol was close to 100%. The activity of each catalyst was analyzed as mmol of product per proton per hour, mmol of product per gram of dry catalyst per hour and mmol of product per cc of wet catalyst per hour. The results are shown in Table 2.

The results show that catalyst A has the highest activity on a per proton basis out of the group of catalysts analyzed. This is understandable since it has the lowest DVB content 7% compared to the others with 12% DVB and therefore the reactants can gain ready access to all the sites. In one variant of the invention, it is useful to have the catalysts with a DVB content in the range of 1-30 percent. On a dry weight basis, catalyst D has the highest activity owing to a high acid site density per gram (see Table1). On a volume basis, catalyst A has the best performance characteristics followed by catalyst D.

TABLE 2

| RESIN | Mmol/H+/hr | Mmol/g/hr | Mmol/cc/hr |
|---|---|---|---|
| A | 4.0 | 10.9 | 4.1 |
| B | 2.4 | 7.8 | 2.9 |
| C | 2.8 | 8.8 | 3.0 |
| D | 2.4 | 11.7 | 3.4 |
| E | 2.1 | 8.8 | 2.2 |

Example 1

Polymer synthesis:

Styrene DVB co-polymers used to make catalysts of the present invention are commercially available in sulfonated forms. For example, Amberlyst 39 (commercially available from Rohm and Haas Company) was chlorinated

to make resin **A** using Scheme 1. Similarly, Amberlyst 16 was used to prepare resin **F**. Treatment of resin **F** with water at 150 degrees C for ten days according to US patent no. 4,705,808 produced resin **C**. Amberlyst 36 was chlorinated to make resin **D** according to Scheme 2. For resin **E**, the substrate 4-chlorostyrene co-polymer used to make a catalyst of the present invention was according to art procedures (British Patent 1,393,594). The sulfonation was done using oleum at 110C for 1 hour - 100g of polymer requires 1000g of oleum.

Example 2

Chlorination of polymer:

The following procedure is representative for all examples and chlorinations. However, it is appreciated that other chlorination procedures can be used to obtain the properties of the catalyst described herein: In a two liter glass reactor connected to a water circulation bath is placed 490 ml of wet Amberlyst 36. To this is added 1180 g of water , and after stirring the water circulation bath is set to 35C. The system is purged out for ten minutes with house nitrogen and then chlorine is introduced into the reactor at 15psig. The chlorine feed rate is a function of reaction rate and can be monitored by measuring weight loss of lecture bottle. The percent HCl in the liquid is a measure of how much chlorine is on the resin since each Cl atom on the resin produces one HCl which dissolves in the water. This serves as a tool to gauge how much chlorine is on the resin. Aliquots of solution can be removed and titrated with base to measure percentage HCl in solution. After 15 hours, the percentage HCl was found to be 2 percent. The reaction was stopped and the resin is washed with several rinses of DI water then sent for analysis. Found Cl : 11.87%, S: 16.66%

Example 3

The following experiment illustrates that base treatment removes leachable chlorine: 75 grams of wet resin F was mixed with 220ml of 50% NaOH and heated for 4 hours at 130C. The resin was filtered and the filtrate plus washes were weighed. A 10 g sample of the filtrate was treated with nitric acid to a pH of 2, then titrated with silver nitrate. Based on this result the amount of chlorine in 10gm of filtrate was normalized to the overall weight of filtrate plus washes. The amount of leachable chlorine in 75 g of wet resin was determined as 1.46g. The same experiment when repeated at room temperature for ten minutes gave only 0.47g of chlorine showing that the heat treatment with base removes additional chlorine from the polymer.

Example 4

The following base treatment procedure was used to make resins **A, B, D, E**. Approximately 275 grams of chlorinated resin (washed with 1500 ml of deionized water) was placed in a 3 neck round-bottomed flask equipped with a mechanical stirrer, water condensor and a Thermowatch. To this was added 1192 ml of a 2N NaOH solution. The mixture was stirred and heated to reflux (103 degrees C) for a total of 22 hrs. The solution was separated from the resin and combined with a 100 ml washing of the resin. The solution was acidified with $HNO_3$ and then titrated with silver nitrate for chloride determination. The resin was washed 3 times with 500 ml of deionized water then transferred to a column and washed with 3 liters of water (3 hrs) and 3 liters of 4% hydrochloric acid (3hrs) and then three liters of water(3hrs).

For resin **A** - the following was found before base treatment: 29.7% Cl, 8.2% S. After base treatment, the following results were obtained: 26.78% Cl, 8.62% S. The calculated amount of chloride recovered from the resin is 6.79g which is consistent with the reported analysis.

For resin **D** - the following was found before base treatment: 11.87% Cl, 16.66% S. After base treatment, the following results were obtained: 10.04% Cl, 16.69% S.

For resin **E** - the following was found before base treatment: 18.87% Cl, 13.97% S. After base treatment, the following results were obtained: 15.96% Cl, 13.95% S.

Example 5

The following example gives one variant of a thermal stability test procedure used herein: In a 125 ml acid digestion bomb is added 40 ml of resin and 28 ml of deionized water. The bomb is sealed and placed in a vacuum oven which is then heated to 200 degrees C. After 24 hours, the heat is turned off and the oven is cooled for 4 hours. The bomb is removed and the lid is removed in a hood. The liquid is separated from the resin and the pH is measured and the chlorine content determined by titration. The resin is washed thoroughly and its acid capacity is measured as described in example 6. The same experiment was also repeated in three smaller digestion bombs, each containing 14 ml of resin and 10 ml of water. After the thermal stability experiment the three samples were combined for analysis.

Results are shown in Table 1.

Example 6

The following procedure is a general method used to determine acid capacity of all resins. The acid capacity of resin can be determined as follows: The wet resin was placed in a beaker and dried overnight at 110 degrees C. The beaker was placed in a dessicator allowed to cool and weighed. The first weight observed on the balance was recorded (allowing the resin to sit exposed to the air results in re-absorption of moisture). The weight of dry solid was recorded (typically we used between 5.5 and 6.0g). After transferring to a column the resin, approximately 300 ml of deionized water was passed through the resin for half hour. Separately, a solution of sodium nitrate was prepared by dissolving 45 g in a liter of water. This was flown through the column in 2 hours and the eluate was collected in a 1000 ml volumetric flask up to the mark. A 100 ml of this sample was titrated with 0.1008 N solution of sodium hydroxide. The acid capacity was calculated as [V1*0.1008*10/W1] mmol H+ per gram of dry resin where V1 is the volume of base required for neutralization and W1 is the dry weight of resin.

Example 7

The following evaluation of the resins created herein as catalysts for butene hydration was performed: The experimental setup used is based on batch type EAZY SEEL autoclave commercially available from Autoclave Engineers. The reactor cup was equipped with a extraction adapter allowing for the taking samples during the reaction at minimum dead volume or quantitative extraction of the entire reaction mixture for "multiple use" sequences. The butene-1 substrate was metered in the calibrated flask and injected into the reactor under 60psi positive pressure. The reaction temperature, the reactor pressure, the ballast tank pressures and the stirring rate were continuously monitored and recorded. A typical hydration charge consisted of 1g catalyst, 10cc butene-1 and 10 cc water. After pressurizing the rector with argon to 550psi the reactor was heated to the target temperature of 155 °C and the stirring initiated of 1200RPM. The reaction continued for the pre-determined amount of time, the reactor was cooled to ambient temperature, and the liquid phase released into a 25cc volumetric flask. After diluting to the mark with dioxane, the reaction mixture was analyzed for the product sec-butyl alcohol. The analysis was done using a GC equipped with a stabiliwax column and an FID with N,N- dimethylformamide as an internal standard. Results are shown in Table 2.

The DVB used herein can be in the range of 63 to 100 percent purity. The DVB obtained commercially for use in the invention can also use alone or in combination with EVB (ethylvinylbenzene), which can also be used in combination with DVB.

While only a few, preferred embodiments of the invention have been described hereinabove, those of ordinary skill in the art will recognize that the embodiment may be modified and altered without departing from the central spirit and scope of the invention. Thus, the preferred embodiment described hereinabove is to be considered in all respects as illustrative and not restrictive, the scope of the invention being indicated by the appended claims, rather than by the foregoing description, and all changes which come within the meaning and range of equivalency of the claims are intended to be embraced herein.

## Claims

**1.** A method of olefin hydration using a catalyst, comprising: hydrating said olefin using a base treated, sulfonated, halogenated and acid regenerated thermally stable catalyst, said catalyst comprising a styrene DVB copolymer, or a styrene DVB/EVB copolymer, or a combination thereof.

**2.** The method of claim 1 in which said olefin hydration is selected from the group consisting of butene hydration, propene hydration, hydration of cyclohexene, propylene hydration, pentene hydration, hexene hydration, and heptene hydration.

**3.** A system for olefin hydration utilizing the method of claim 1.

**4.** An alcohol manufactured utilizing the method of claim 1.

**5.** A method of making a thermally stable catalyst for olefin hydration, comprising:

(a) base treating a sulfonated and halogenated copolymer to obtain a base treated copolymer, and regenerating said base treated copolymer with an acid;

or

(b) base treating a halogenated copolymer to obtain a base treated copolymer, and sulfonating said base treated copolymer;

or

(c) base treating a sulfonated and halogenated copolymer to obtain a base treated copolymer, and regenerating said base treated copolymer with an acid;

or

(d) chlorinating a styrene DVB copolymer, base treating said halogenated copolymer, and sulfonating said halogenated and base treated catalyst,

in which said catalyst comprises a styrene DVB copolymer, or a styrene DVB/EVB copolymer, or a combination thereof.

**6.** The method of claim 5 in which leachable chlorine is removed from a polymer backbone of said catalyst by said base treatment.

**7.** The method of claim 5 in which said styrene DVB copolymer is a polysulfonated copolymer.

**8.** An improved styrene DVB resin catalyst, an DVB/EVB resin catalyst, or a combination thereof, comprising aromatic groups having more than one $SO_3H$ moiety and a polymer backbone, said improvement comprising halogenated aromatic groups, said polymer backbone being substantially free of leachable chlorine, and said resin capable of olefin hydration.

**9.** An improved styrene DVB resin catalyst, a styrene DVB/EVB resin catalyst, or combination thereof comprising 4-halostyrene aromatic groups and a polymer backbone, at least one of said aromatic groups having a chlorine in a styrenic para-position, and optional other chlorines thereon; said polymer backbone being substantially free of leachable chlorine, and, a halogenated DVB moiety or a halogenated DVB/EVB moiety.

**10.** The improved catalyst of claim 9 in which said aromatic rings are oleum sulfonated.

**11.** The improved catalyst of claim 9 further comprising sulfone bridges.

European Patent Office

# EUROPEAN SEARCH REPORT

Application Number
EP 04 25 2612

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | US 4 025 467 A (BROCK HERBERT B) 24 May 1977 (1977-05-24)<br>* column 1, line 47 - column 2, line 42 *<br>* example 1 *<br>----- | 1-11 | C07C29/04<br>B01J31/10<br>B01J37/06 |
| D,A | US 4 705 808 A (NEIER WILHELM ET AL) 10 November 1987 (1987-11-10)<br>* the whole document *<br>----- | 1-11 | |
| A | US 4 358 626 A (OKUMURA YOSHIHARU ET AL) 9 November 1982 (1982-11-09)<br>* examples 1-8 *<br>----- | 1-11 | |
| A | US 4 340 769 A (WOELLNER JOHANNES ET AL) 20 July 1982 (1982-07-20)<br>* the whole document *<br>----- | 1-11 | |
| A | US 4 595 786 A (WALLER FRANCIS J) 17 June 1986 (1986-06-17)<br>* claim 1 *<br>----- | 1-11 | TECHNICAL FIELDS SEARCHED (Int.Cl.7)<br>C07C<br>B01J |

The present search report has been drawn up for all claims

| Place of search | Date of completion of the search | Examiner |
|---|---|---|
| The Hague | 23 September 2004 | O'Sullivan, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 04 25 2612

This annex lists the patent family members relating to the patent documents cited in the above-mentioned European search report.
The members are as contained in the European Patent Office EDP file on
The European Patent Office is in no way liable for these particulars which are merely given for the purpose of information.

23-09-2004

| Patent document cited in search report | Publication date | Patent family member(s) | Publication date |
|---|---|---|---|
| US 4025467 A | 24-05-1977 | CA 1049190 A1 | 20-02-1979 |
| | | DD 125584 A5 | 04-05-1977 |
| | | DE 2625683 A1 | 30-12-1976 |
| | | FR 2314213 A1 | 07-01-1977 |
| | | GB 1547831 A | 27-06-1979 |
| | | IT 1062331 B | 10-10-1984 |
| | | JP 1693809 C | 17-09-1992 |
| | | JP 2042542 B | 25-09-1990 |
| | | JP 51151286 A | 25-12-1976 |
| | | NL 7606377 A | 15-12-1976 |
| | | SE 431219 B | 23-01-1984 |
| | | SE 7606115 A | 14-12-1976 |
| | | SU 644390 A3 | 25-01-1979 |
| | | ZA 7603302 A | 27-07-1977 |
| US 4705808 A | 10-11-1987 | DE 3512517 A1 | 09-10-1986 |
| | | AT 64695 T | 15-07-1991 |
| | | AU 574572 B2 | 07-07-1988 |
| | | AU 5558186 A | 09-10-1986 |
| | | BR 8601564 A | 09-12-1986 |
| | | CA 1263372 A1 | 28-11-1989 |
| | | CH 667018 A5 | 15-09-1988 |
| | | CN 86102001 A ,B | 01-10-1986 |
| | | CS 8601993 A2 | 14-04-1989 |
| | | DD 247156 A5 | 01-07-1987 |
| | | DE 3679930 D1 | 01-08-1991 |
| | | DK 154886 A ,C | 07-10-1986 |
| | | EP 0198270 A2 | 22-10-1986 |
| | | ES 8701522 A1 | 01-03-1987 |
| | | FI 861381 A ,B, | 07-10-1986 |
| | | IN 167216 A1 | 22-09-1990 |
| | | JP 1734128 C | 17-02-1993 |
| | | JP 4025066 B | 28-04-1992 |
| | | JP 61230744 A | 15-10-1986 |
| | | MX 168180 B | 10-05-1993 |
| | | NO 861013 A ,B, | 07-10-1986 |
| | | PL 145847 B1 | 30-11-1988 |
| | | RO 95285 A1 | 15-09-1988 |
| | | SU 1443804 A3 | 07-12-1988 |
| | | TR 22885 A | 03-10-1988 |
| | | YU 49586 A1 | 30-06-1988 |
| | | ZA 8602535 A | 26-11-1986 |
| US 4358626 A | 09-11-1982 | JP 1331708 C | 14-08-1986 |
| | | JP 57108025 A | 05-07-1982 |
| | | JP 60059217 B | 24-12-1985 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO. EP 04 25 2612

This annex lists the patent family members relating to the patent documents cited in the above-mentioned European search report.
The members are as contained in the European Patent Office EDP file on
The European Patent Office is in no way liable for these particulars which are merely given for the purpose of information.

23-09-2004

| Patent document cited in search report | Publication date | Patent family member(s) | Publication date |
|---|---|---|---|
| US 4358626 A | | CA 1188711 A1 | 11-06-1985 |
| | | DE 3171618 D1 | 05-09-1985 |
| | | EP 0055522 A1 | 07-07-1982 |
| US 4340769 A | 20-07-1982 | DE 2233967 A1 | 31-01-1974 |
| | | AR 196934 A1 | 28-02-1974 |
| | | AT 326091 B | 25-11-1975 |
| | | AT 476373 A | 15-02-1975 |
| | | AU 477327 B2 | 21-10-1976 |
| | | AU 5755273 A | 09-01-1975 |
| | | BE 802139 A1 | 10-01-1974 |
| | | CA 995256 A1 | 17-08-1976 |
| | | CS 179419 B2 | 31-10-1977 |
| | | DD 106818 A5 | 05-07-1974 |
| | | DK 152489 B | 07-03-1988 |
| | | ES 415669 A1 | 16-01-1976 |
| | | FI 60193 B | 31-08-1981 |
| | | FR 2192089 A1 | 08-02-1974 |
| | | GB 1396488 A | 04-06-1975 |
| | | IN 138893 A1 | 10-04-1976 |
| | | IT 998198 B | 20-01-1976 |
| | | JP 49051211 A | 18-05-1974 |
| | | JP 58007614 B | 10-02-1983 |
| | | NL 7309668 A ,B, | 15-01-1974 |
| | | NO 141012 B | 17-09-1979 |
| | | RO 72492 A1 | 11-10-1982 |
| | | SE 387105 B | 30-08-1976 |
| | | SU 512695 A3 | 30-04-1976 |
| | | YU 158873 A1 | 27-04-1983 |
| | | ZA 7304502 A | 31-07-1974 |
| US 4595786 A | 17-06-1986 | NONE | |

EPO FORM P0459

For more details about this annex : see Official Journal of the European Patent Office, No. 12/82